# EUROPÄISCHE PATENTSCHRIFT

(11) **EP 0 796 848 B1**
(45) Veröffentlichungstag und Bekanntmachung des Hinweises auf die Patenterteilung: **04.06.2003**
(21) Anmeldenummer: 97103611.6
(22) Anmeldetag: 05.03.1997
(51) Int. Cl.: C07D 215/26, A61K 31/47

(54) **Fluoralkyl- und Fluoralkoxysubstituierte hetero-cyclische Bradykinin-Antagonisten**
Fluoralkyl- and fluoralkoxy-substituted heterocyclic bradykinin antagonists
Bradykinine antagonistes hétérocycliques substituées par fluoralkyl ou par fluoralkoxy

(30) Priorität: 19.03.1996 DE 19610784
(43) Veröffentlichungstag der Anmeldung: 24.09.1997
(73) Patentinhaber: HOECHST AKTIENGESELLSCHAFT, 65929 Frankfurt am Main (DE)
(72) Erfinder: Wagner, Adalbert, Dr., 86368 Gersthofen (DE); Heitsch, Holger, Dr., 55252 Mainz-Kastel (DE); Nölkne, Gerhard, Dr., 65843 Sulzbach (DE); Wirth, Klaus, Dr., 65830 Kriftel (DE); Bernard, Prof,-Dr., 65779 Kelkheim (DE)

(56) Entgegenhaltungen:
- EP-A- 0 622 361

## Beschreibung

Die Erfindung betrifft heterocyclische fluoralkylsubstituierte und fluoralkoxysubstituierte Verbindungen mit bradykinin-antagonistischer Wirkung.

Aus EP-A 622 361, US 5,212,182, US 5,216,165 und US 5,438,064 sind O-und N-substituierte Chinoline und ihre Verwendung als Bradykinin-Rezeptorantagonisten bekannt.

Überraschenderweise wurde gefunden, daß die Einführung von Fluoralkylgruppen Verbindungen liefert, die eine deutlich längere Wirkdauer aufweisen.

Die vorliegende Erfindung betrifft heterocyclische Fluoralkylderivate und Fluoralkoxyderivate der Formel (I) 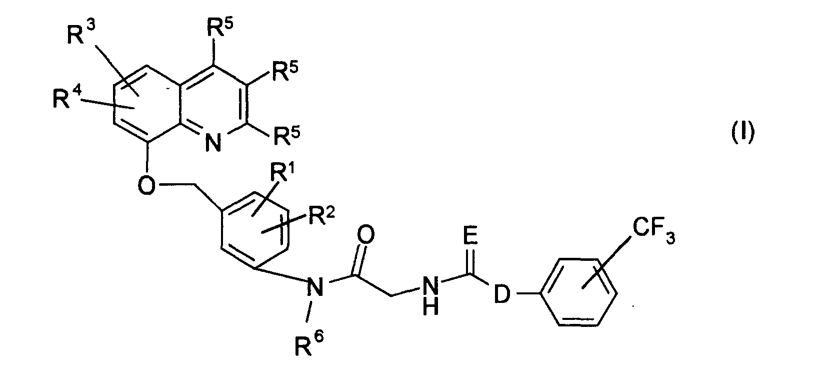 in welcher
- R¹ und R²: Chlor,
- R³ und R⁴: gleich oder verschieden H oder (C₁-C₃)-Alkyl
- R⁵: H oder CH₃,
- R⁶: (C₁-C₅)-Alkyl,
- D: (C₁-C₅)-Alkandiyl oder (C₂-C₅)-Alkendiyl, und
- E: O oder S bedeuten,
sowie deren physiologisch verträglichen Salze.

Alkyl und Alkenyl können geradkettig oder verzweigt sein. Entsprechendes gilt für davon abgeleitete Reste wie z. B. Alkoxy.

Unter physiologisch verträglichen Salzen von Verbindungen der Formel (I) versteht man sowohl deren organische als auch anorganische Salze, wie sie in Remington s Pharmaceutical Sciences (A.R. Gennard (Editor), Mack Publishing Co., Easton PA, 17. Auflage, Seite 1418 (1985)) beschrieben sind. Aufgrund der physikalischen und chemischen Stabilität und der Löslichkeit sind als saure Gruppen unter anderem Natrium-, Kalium-, Calcium- und Ammoniumsalze bevorzugt; als basische Gruppen sind unter anderem Salze der Salzsäure, Schwefelsäure, Phosphorsäure oder von Carbonsäuren oder Sulfonsäuren, wie z. B. Essigsäure, Zitronensäure, Benzoesäure, Maleinsäure, Fumarsäure, Weinsäure und p-Toluolsulfonsäure bevorzugt.

Die Erfindung betrifft weiterhin ein Verfahren zur Herstellung von Verbindungen der Formel (I), das dadurch gekennzeichnet ist, daß man
a) eine Verbindung der Formel (II), 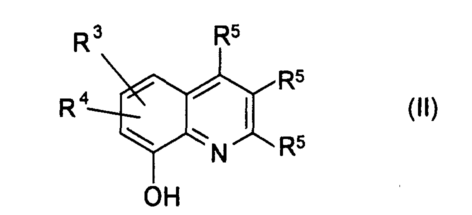 worin R³, R⁴ und R⁵ wie oben in Formel (I) definiert sind, mit CS₂CO₃ oder K₂CO₃ in einem inerten Lösungsmittel, bevorzugt DMF oder N-Methylpyrrolidin, deprotoniert und bei Raumtemperatur mit einer Verbindung der Formel (III) 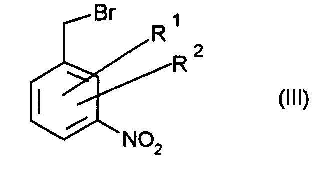 worin R¹ und R² wie oben in Formel (I) definiert sind, umsetzt;
b) die so erhaltene Verbindung der Formel (IV) 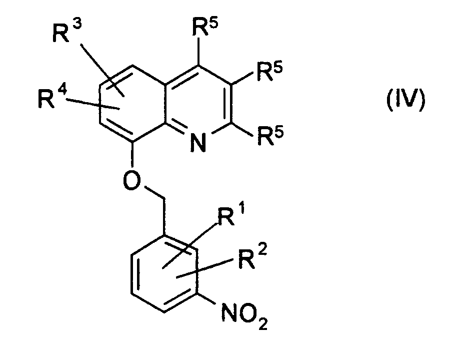 worin R¹, R², R³, R⁴ und R⁵ wie oben in Formel (I) definiert sind, mit Hilfe von Übergangsmetallhalogeniden, bevorzugt SnCl₂, FeCl₃ zu einer Verbindung der Formel (V) reduziert, 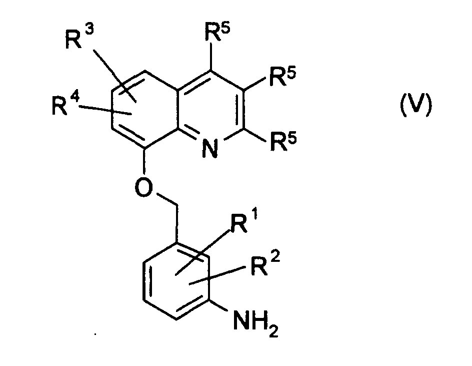 worin R¹, R², R³, R⁴, und R⁵ wie oben in Formel (I) definiert sind;
c) eine Verbindung der Formel (V) mit aktivierten, geeignet geschützten Glycin (-C(O)-(CH₂)-N(Prot)), in inerten Lösungsmitteln, gegebenenfalls durch Zugabe von DMAP, umsetzt und so eine Verbindung der Formel (VI) erhält, 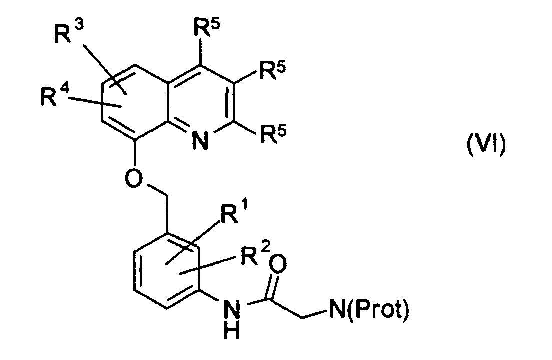 worin R¹, R², R³, R⁴, und R⁵ wie oben in Formel (I) definiert sind, und Prot für eine Aminoschutzgruppe steht, wie in T.W. Greene Protective Groups in organic Synthesis, Verlag John Wiley, 2. Auflage 1991 beschrieben, z.B. Phthaloyl, Benzyl oder Paramethoxybenzyl;
d) eine Verbindung der Formel (VI) nach erfolgter Einwirkung von Alkalihydriden, Alkalicarbonaten oder Alkoholaten in inerten Lösungsmitteln, bevorzugt DMF oder NMP, gefolgt von einer Behandlung mit R⁶X, wobei R⁶ wie oben in Formel (I) definiert ist und X eine Abgangsgruppe, z. B. Halogen, Mesylat oder Tosylat, bedeutet, umsetzt, wobei man eine Verbindung der Formel (VII) erhält, 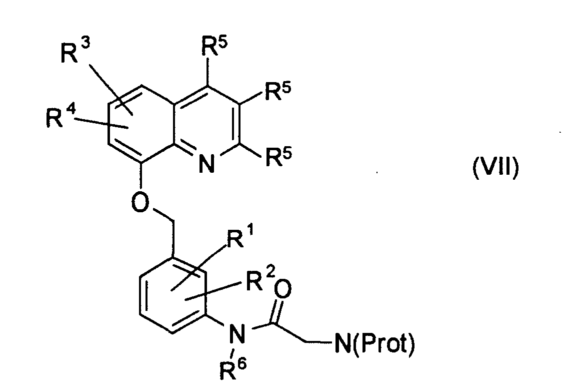 worin R¹, R², R³, R⁴, R⁵ und R⁶, wie oben in Formel (I) und Prot wie oben in Formel (VI) definiert sind;
e) zum Entfernen der Schutzgruppe (Prot) von der Verbindung der Formel (VII), im Falle der Phthaloylgruppe bevorzugt mit Hydrazin in Alkoholen als Lösungsmittel bei Temperaturen zwischen Raumtemperatur und dem Siedepunkt, bevorzugt bei Raumtemperatur, umsetzt wobei man eine Verbindung der Formel (VIII) erhält, 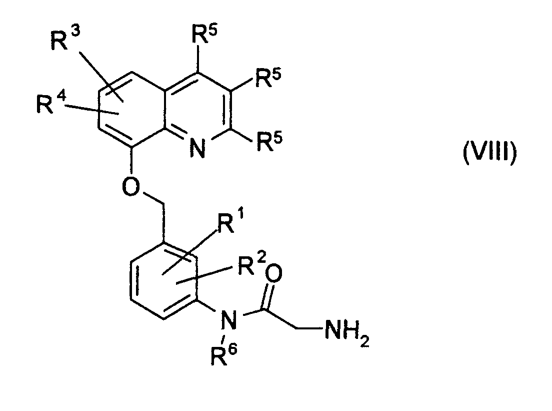 worin R¹, R², R³, R⁴, R⁵ und R⁶ wie oben in Formel (I) und Prot wie oben in Formel (VI) definiert sind;
f₁) eine Verbindung der Formel (VIII) mit aktivierten Carbonsäurederivaten der Formel (IX), 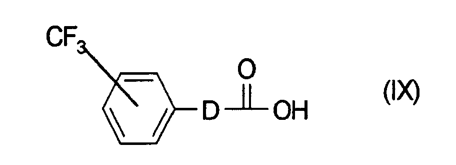 worin D wie oben in Formel (I) definiert sind,
   bevorzugt deren Säurechloriden oder Carbonsäuren der Formel (IX), aktiviert durch Reagenzien, wie sie in der Peptidsynthese verwendet werden, umsetzt, oder
f₂) eine Verbindung der Formel (VIII) mit einem Amin oder einem Alkohol der Formel (X) 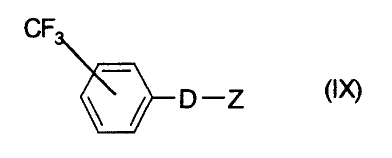 worin D wie oben definiert sind und Z OH oder NH₂ bedeutet, wobei man zunächst jedoch die Verbindung der Formel (VIII) oder (X) mit einer doppelt aktivierten Carbonylverbindung zur Bildung der Harnstoff- bzw. Urethangruppe, z.B. mit Carbodiimiden, Phosgen oder Chlorkohlensäureestern, bevorzugt Phosgen und Carbonyldiimidazol, reagieren läßt, umsetzt, bevorzugt bei Temperaturen zwischen 0 C und Raumtemperatur in inerten Lösungsmitteln, bevorzugt Dichlormethan oder Dimethoxyethan, oder
f₃) eine Verbindung der Formel (VIII) mit einem entsprechenden Isocyanat oder Isothiocyanat, bevorzugt bei Temperaturen zwischen 0°C und Raumtemperatur in inerten Lösungsmitteln, bevorzugt Dichlormethan oder Dimethoxyethan, umsetzt, und
g) die erhaltene Verbindung der Formel (I) gegebenenfalls nach bekannten Methoden in ihre physiologisch verträglichen Salze überführt.

Die Konversion zur Bromethylverbindung erfolgt durch Umsetzung des entsprechenden Methyl-Derivates mit N-Bromsuccinimid, Dibromhydantoin oder Brom in inerten Lösungsmitteln, bevorzugt Brombenzol oder Cyclohexan bei Temperaturen von 60 °C bis zum Siedepunkt.

Als Kupplungsreagenz können alle möglichen in der Peptidsynthese verwendeten Aktivierungsreagenzien, siehe z. B. Houben-Weyl, Methoden der organischen Chemie, Band 15/2, Georg Thieme Verlag, Stuttgart 1974, verwendet werden, insbesondere aber Carbodiimide wie z. B. N,N'-Dicyclohexylcarbodiimid, N,N'-Diisopropyl-carbodiimid oder N-Ethyl-N'-(3-dimethylaminopropyl)carbodiimid. Die Kupplung kann dabei direkt durch Addition von Carbonsäurederivat mit dem Aktivierungsreagenz und gegebenenfalls einem Zusatz wie z.B. 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R.Geiger, Chem.Ber. 103, 2054 (1970)) durchgeführt werden oder aber die Voraktivierung des Carbonsäurederivats als symmetrisches Anhydrid oder HOBt- bzw. HOObt-Ester kann separat erfolgen und die Lösung der aktivierten Spezies in einem geeigneten Lösungsmittel zum Amin gegeben werden.

Die Kupplung bzw. Aktivierung der Aminosäurederivate mit einem der oben genannten Aktivierungsreagenzien kann in Dimethylformamid, N-Methylpyrrolidon oder Methylenchlorid oder einer Mischung aus den genannten Lösungsmitteln durchgeführt werden.

Anstelle der Phthaloylgruppe können auch Schutzgruppen verwendet werden, die beide Protonen der Aminogruppe schützen, z.B. 2 Benzyl-Gruppen.

Die erfindungsgemäßen Verbindungen haben einzeln oder in Kombination eine bradykinin-antagonistische Wirkung, die in verschiedenen Modellen getestet werden kann (s. Handbook of Exp. Pharmacol. Vol. 25, Springer Verlag, 1970, S. 53 - 55), so z. B. am isolierten Rattenuterus, am Meerschweinchenileum, der Jugularvene des Kaninchens oder an der isolierten Pulmonalarterie des Meerschweinchens. Die Effekte der Verbindungen der Formel (I) auf bradykinininduzierte Bronchokonstriktion und Carrageenin induziertes Pfotenödem können analog Br. J. Pharmacol. 102, 774 - 777 (1991) bestimmt werden.

Die Messung der Bindung an den Bradykinin B₂ Rezeptor vom Meerschweinchenileum ist im Folgenden beschrieben (R.B.Innis et al., Proc.Natl.Acad.Sci.USA; 17 (1981) 2630):

### 1. Ligand: ³H-BRADYKININ (von NEN Du Pont)

### 2. Pufferansätze:

### a) TES-Puffer:

25 mM TES (SIGMA, Best.Nr.: T-4152)
1 mM 1,10-Phenanthrolin (SIGMA; Best.Nr.: P-9375)

### b) Inkubations-Puffer:

25 mM TES (SIGMA; Best.Nr.: T-4152)
1 mM1,10-Phenanthrolin (SIGMA; Best.Nr.: P-9375)
0,1% Albumin, Bovine (SIGMA; Best.Nr.: A-7906)
140 µg/ml Bacitracin (SIGMA; Best.Nr.: B-0125)
1 mM Dithiothreitol (SIGMA; Best.Nr.: D-0632)
1 µM Captopril - 1-[(2S)-3-Mercapto-2-methylpropionyl]-L-prolin

Beide Puffer werden mit 5 molarer NaOH auf pH 6,8 eingestellt.

### 3. Membranpräparation:

Meerschweinchen-Ilea werden durch vorsichtiges Ausstreichen grob vom Darm-Inhalt befreit und in 0,9%iger NaCI-Lösung gesäubert.
Die ca. 2 cm langen llea-Stücke werden in eiskalten TES-Puffer überführt (ca. 1g /10 ml) und mit dem Ultraturrax ca. 30 sec. lang im Eisbad homogenisiert. Das Homogenat wird anschließend durch 3 Lagen Gaze gefiltert und das Filtrat bei 50.000 g 10 Minuten zentrifugiert.
Der Überstand wird verworfen, das Pellet in dem gleichem Volumen TES-Puffer rehomogenisiert und erneut bei 50.000 g 10 Minuten zentrifugiert.
Das Pellet wird in Inkubations-Puffer rehomogenisiert (ca. 1g / 5 ml) und in Kryoröhrchen, 2 ml portioniert, bei -70°C eingefroren.

Die Proteinkonzentration der fertigen Membransuspension wird nach LOWRY bestimmt und sollte ca. 15 µg / 100 µl betragen.

### 4. Bindungstest:

Alle Inkubationen werden bei Raumtemperatur für 60 Minuten auf Mikrotiterplatten (96 x 300 µl) in 200 µl Volumen durchgeführt. Alle Ansätze in Inkubations-Puffer. Dazu werden 50 µl des Radioliganden, 50 µl des zu prüfenden Präparats und 100 µl der Membransuspension nacheinander in die Vertiefungen der Mikrotiterplatte pipettiert.

### a) Sättigungsexperimente (Heiße Sättigung):

Herstellung der ³H-Bradykinin-Lösung: Für die Sättigungsexperimente werden die Konzentrationen 0.05, 0.1, 0.2, 0.4, 0.6, 0.8, 1.0, 1.5, 2.0, 2.5 und 3.0 nMol/l, das entspricht 0.05 bis 3.0 pMol/ml, eingesetzt. Nach der Herstellung der entsprechenden Verdünnungen werden je 50 µl pro Probe vorgelegt.

Unspezifische Bindung: Für jede Konzentration des radioaktiven Liganden muß die unspezifische Bindung bestimmt werden. Dies kann durch Zugabe einer hohen Konzentration (1-100 µMol) des nichtmarkierten Liganden, anderer Antagonisten oder Agonisten des Bradykinin-Rezeptors erreicht werden. In diesem Test wird HOE 140 (10 µMol/l) verwendet. Dazu werden 1,862 mg in 1 ml Dimethylsulfoxid (DMSO) gelost, 1:25 mit Inkubationspuffer verdünnt und von dieser Lösung 50 µl zu den Proben in die Mikrotiterplatte gegeben. Die Reaktion wird durch die Zugabe von 100 µl der Membransuspension gestartet.

### b) Kompetitionsexperimente (IC₅₀):

Hier werden eine feste Größe des radioaktiven Liganden (0,25 bis 0,3 nMol/l ³H-Bradykinin) und verschiedene Konzentrationen der nichtmarkierten Agonisten oder Antagonisten eingesetzt.
Zu jeweils 50 µl der ³H-Bradykinin-Lösung werden 50 µl der zu prüfenden Präparate bzw. Standards in den Konzentrationen 10⁻⁵ bis 10⁻¹⁰ Mol/l zugegeben und die Reaktion durch Zugabe von 100 µl Membransuspension gestartet. Auch in diesem Test werden 3fach-Bestimmungen durchgeführt und drei Proben zur Bestimmung der unspezifischen Bindung mit 10 µMol/l HOE 140 inkubiert.

Die auf Kompetition zu prüfenden Präparate werden grundsätzlich in einer Konzentration von 1 mMol/l in Dimethylsulfoxid (DMSO) gelöst, und anschließend mit DMSO weiterverdünnt. Diese Lösung wird dann 1:25 mit Inkubationspuffer verdünnt.

Nach der Inkubation werden die Proben im Skatron Zellharvester über einen vorher mit 0,1% PEI (Polyethylenimin) angefeuchteten Whatmann GF/B Filterpapierstreifen abfiltriert und mit, je Probe, 10 ml eiskaltem TES-Puffer nachgewaschen. Die noch feuchten Filter werden in Mini-Scintillationsröhrchen ausgestanzt und mit 3 ml Scintillator aufgefüllt.
Nach ca. 12 Stunden Einweichzeit werden die Proben kurz aufgeschüttelt und im Beta-Counter gemessen.

### c) Screening:

Im Primär-Screening werden im allgemeinen nur 1-2 Konzentrationen des Prüfpräparats (10⁻⁵ und 10⁻⁶ Mol/l) eingesetzt. Ist bei der höchsten Konzentration eine Verdrängung des Radioliganden von 50% oder mehr nachweisbar, wird eine vollständige Analyse (Kompetitions-Experiment) mit mind. 8 Konzentrationen vorgenommen.

### 4. Auswertung:

Die Auswertung erfolgt mittels des LIGAND-Programmpakets (Mc Pherson, Minson & Rodbard, Vertrieb: Elsevier-BIOSOFT), das die notwendigen Berechnungen zur Bestimmung von IC₅₀ und Kᵢ-Werten vornimmt. Dieses Programm führt außerdem grafische Darstellungen der Sättigungs- bzw. Verdrängungs-Kurven sowie den SCATCHARD-Plot, HILL-Plot oder HOFSTEE-Plot durch.

### 5. Testergebnisse:

Nach dem oben angeführten verfahren wurden für die Verbindungen der Beispiele 1, 2, 8, 22 und 29 als repräsentative Verbindungen der beschriebenen Fluoralkyl- und Fluoralkoxysubstituierten heterocyclischen Bradykinin-Antagonisten der Formel (I) folgende IC₅₀- und Kᵢ-Werte bestimmt:

| Beispiel | IC₅₀ [nM] | Kᵢ [nM] |
|---|---|---|
| 1 | 9.0 | 1.0 |
| 2 | 40.0 | 4.0 |
| 8 | 28.0 | 3.4 |
| 22 (Vgl.) | 95.0 | 10.0 |
| 29 (Vgl.) | 38.0 | 5.1 |

Die Bestimmungen der antagonistischen Wirkung auf die bradykinininduzierte Kontraktion des Meerschweinchen-lleums erfolgt nach folger dem Protokoll:

Meerschweinchen im Gewicht von ca. 300 g (Morioth strain,♂♀) werden durch Nackenschlag getötet und entblutet. Das Ileum wird in einer Länge von ca. 20 cm herauspräpariert, mit Tyrode-Lösung durchspült (Recordspritze) und so vom Darminhalt befreit. Nun wird es in 1,5 cm lange Abschnitte geteilt. Diese werden in 10 ml fassenden Organbädern, die mit Tyrode-Lösung gefüllt sind, fixiert und mit Dehnungsmeßstreifen (isometrische Kontraktionsmessung) verbunden. Die Vorlast beträgt 1g. Die Tyrode-Lösung wird in einem Wasserbad auf 37°C erwärmt und mit Druckluft durchperlt.
Nach einem Intervall von 30 min. wird mit dem Versuch begonnen.
Nach Aufzeichnung der biologischen Null-Linie wird pro Organbad Bradykinin in einer Endkonzentration von 4 x 10⁻⁸ mol/l zugesetzt und die Konzentration aufgezeichnet. Danach wird 3 min. mit Tyrode-Lösung gespült und nach einer Ruhepause von 20 min. wieder Bradykinin hinzugefügt. Das Maximum der Kontraktion ist erreicht (Kontrolle). Wieder spülen, Ruhepause. Nun wird der Bradykinin-Antagonist zugefügt (Einwirkungszeit 10 min.). Danach wird wieder Bradykinin zugesetzt und die nun erfolgende Kontraktion mit der Kontrolle verglichen. Die Aufzeichnung des Versuchs erfolgt auf einem Tintenschreiber.

| Tyrode-Lösung (mM): | |
|---|---|
| NaCl | 137 |
| Glucose | 5,05 |
| KCl | 2,68 |
| NaHCO₃ | 11,9 |
| NaH₂PO₄ | 0,47 |
| MgCl₂ x 2H₂O | 0,49 |
| CaCl₂ x 2H₂O | 0,68 |

Verstärker: TF6 V3 Fa. Fleck, Mainz
Tintenschreiber: Goerz Metrawatt SE 460, BBC
Bradykinin: Fa. Bachem

So besitzen z. B. die Verbindungen der Beispiele 1 und 2 die folgenden, nach obigen Verfahren ermittelte IC₅₀-Werte:

| Beispiel | IC₅₀ [nM] |
|---|---|
| 1 | 44.0 |
| 2 | 1500.0 |

Die deutlich verlängerte Wirkdauer der Verbindungen der Formel (I) wurde an der Jugularis des Kaninchens nachgewiesen und ist im folgenden beschrieben:

Verglichen wurden die Verbindungen der Beispiele 1, X und Y. Beispiele X und Y sind in EP-A 622 361 beschrieben.

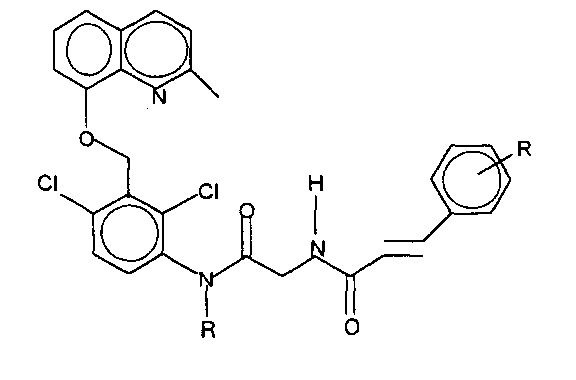

| | |
|---|---|
| Beispiel 1 | 4-CF₃ |
| Beispiel X | 4-CH₃ |
| Beispiel Y | 3-OCH₃ |

Vergleich der in-vitro-Wirkdauer heterozyklischer Bradykinin-Antagonisten an der isolierten Kaninchen-Jugularvene:

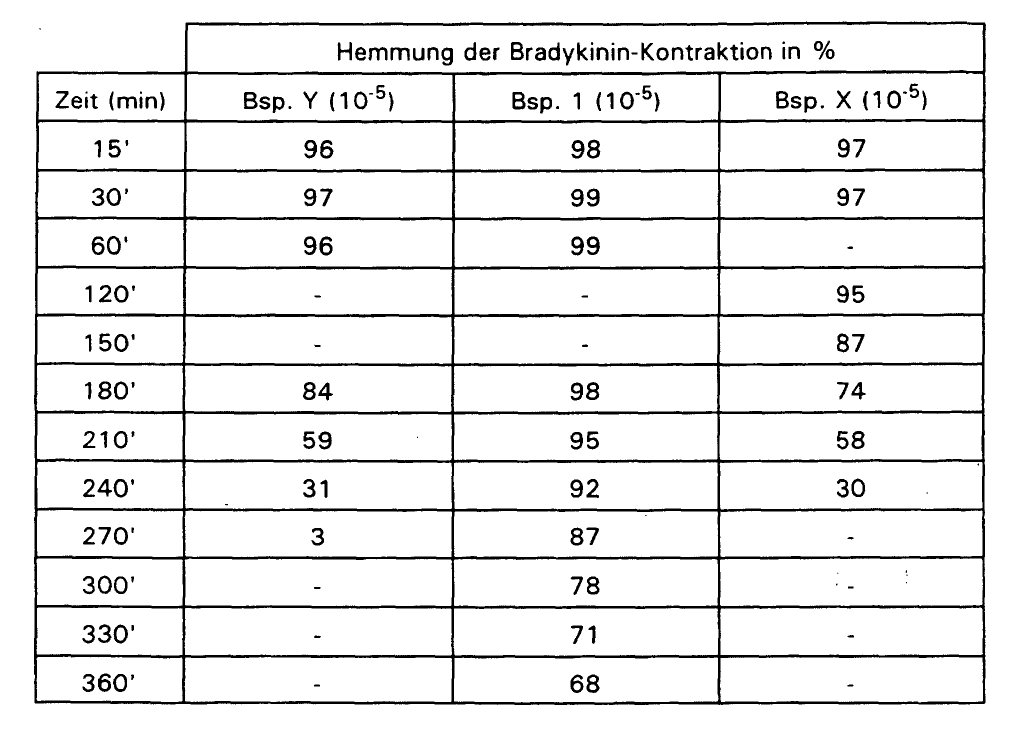

### Methodenbeschreibung:

Männliche Kaninchen (weiße Neuseeländer, Züchter: Möllegaard, Dänemark, 2,5 - 3,0 kg) werden durch Injektion einer Überdosis Pentobarbital-Na (1 ml Narcoren® + 0,5 ml Heparin) getötet. Die beiden Jugularis-Venen werden freipräpariert, spiralförmig aufgeschnitten und Stücke von ca. 1,5 cm länger in gepufferte Organbäder (Krebs-Henseleit-Puffer) bei einer Vorspannung von 0,5 g eingehängt.
Nach 30 min Ruhezeit werden Kontraktionen ausgelöst durch Zugabe von Bradykinin (10⁻⁷ M), die als Ausgangswert dienen. Prüfsubstanzen werden nun in einer Konzentration von 10⁻⁵ M zugegeben. Die gezeigten Hemmwerte sind Mittelwerte (n = 6).
Die angegebenen Werte zum Zeitpunkt 15 min zeigen die Hemmung der Bradykinin-induzierten Kontraktion noch in Anwesenheit der Prüfsubstanzen in der Badflüssigkeit nach 15-minütiger Inkubation. Danach wird die Bradykinin-Kontraktion durch Spülen mit bloßer Pufferlösung beendet.
Zu jedem weiteren aufgeführten Zeitpunkt wurde erneut mit Bradykinin stimuliert (in Abwesenheit der Prüfsubstanz in der Badflüssigkeit) und zur Beendigung der Kontraktion die Badflüssigkeit durch bloße Pufferlösung ersetzt.

### Ergebnisse:

Im Vergleich zu den Verbindungen der Beispiele Y und X zeigt die Verbindung des Beispiels 1 Überlegenheit in Form einer deutlich längeren in-vitro-Wirkdauer. Diese ist ein Maß für die Festigkeit der Bindung an den Rezeptor. Die Verbindungen der Beispiele Y und X zeigen nach 4 h keine Hemmung mehr, während die Verbindung des Beispiels 1 noch nach 6 h mit 68 % starke Hemmungwirkung aufweist.

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Die beschriebenen Verbindungen der Formel (I) und deren pharmakologisch geeigneten Salze sind potente Bradykinin-Antagonisten. Daher liegt ihr therapeutischer Nutzen in der Behandlung und/oder der Prevention aller pathologischer Zustände, die durch Bradykinin und Bradykinin-analoge Peptide vermittelt, ausgelöst oder unterstützt werden. Dies beinhaltet u. a. Allergien, Entzündungen, Autoimmunerkrankungen, Schock, Schmerz und, mehr speziell, Asthma, Husten, Bronchitis, Rhinitis, chronisch obstruktive Pulmonalerkrankungen, Pneumonitis, septischen Schock, endotoxischen Schock, anaphylaktischen Schock, disseminierende intravaskuläre Koagulopathie, Arthritis, Rheuma, Osteoarthritis, Lumbago, entzündungsinduzierte Knochenresorption, Konjunctivitis, Iritis, Kopfschmerz, Migräne, Zahnschmerz, Rückenschmerz, krebsbedingte Schmerzen, postoperativen Schmerz, Traumata (Wunden, Verbrennungen usw.), Ausschlag, Erytheme, Ödeme, Ekzeme, Dermatitis, Zoster, Herpes, Juckreiz, Psoriasis, Flechte, entzündliche Darmerkrankungen, Hepatitis, Pankreatitis, Gastritis, Ösophagitis, Nahrungsallergien, Geschwüre, Reizdarm, Angina, Hirnödem, niedriger Blutdruck, Thrombose, Schädel-Hirn- und Wirbelsäulen-Trauma, Frühgeburt, Atherosklerose, Aszites bei Malignom, Tumormetastasen, Hirnödem bei Tumoren, Hitzeschädigung des Gehirns und Virus-Erkrankungen.

Da weiterhin bekannt ist, daß Bradykinin verknüpft ist mit der Freisetzung von Mediatoren wie Prostaglandinen, Leukotrienen, Tachykininen, Histamin, Thromboxanen, besitzen die Verbindungen der Formel (I) somit auch das Potential zur Behandlung und/oder Prevention von den Krankheiten, die durch diese Mediatoren hervorgerufen werden.

Die Erfindung betrifft daher auch die Verwendung von Verbindungen der Formel (I) als Heilmittel und pharmazeutische Präparate, die diese Verbindungen enthalten.

Pharmazeutische Präparate enthalten eine wirksame Menge des Wirkstoffs der Formel (I) - einzeln oder in Kombination - zusammen mit einem anorganischen oder organischen pharmazeutisch verwendbaren Trägerstoff.

Die Anwendung kann enteral, parenteral - wie z. B. subkutan, i. m. oder i. v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die inhalative Anwendung können Vernebler oder Druckgaspackungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Sollten die Halbwertszeiten der beschriebenen Arzneistoffe in Körperflüssigkeiten unzureichend sein, ist der Einsatz von injizierbaren Retardzubereitungen sinnvoll.
Als Arzneiformen können z. B. ölige Kristallsuspensionen, Mikrokapseln, Rods oder Implantate verwendet werden, wobei die letzteren aus gewebsverträglichen Polymeren, insbesondere bioabbaubaren Polymeren, wie z. B. auf der Basis von Polymilchsäure-Polyglykolsäure-Copolymeren oder Humanalbumin aufgebaut sein können.

Ein geeigneter Dosisbereich für topische und inhalative Anwendungsformen sind Lösungen mit 0,01 - 5 mg/l, bei systemischen Applikationsformen sind 0,01 - 10 mg/kg geeignet.
Allgemein können pro Tag Mengen zwischen 0,1 mg bis zu 1000 mg bezogen auf einen Erwachsenen von 75 kg Körpergewicht appliziert werden.

### Liste der Abkürzungen:

| | |
|---|---|
| AIBN | α, α'-Azobisisobutyronitril |
| DEI | Desorption Electron Impact |
| DCI | Desorption-Chemical Ionisation |
| EE | Ethylacetat |
| FAB | Fast Atom Bombardment |
| DME | Dimethoxyethan |
| DMF | Dimethylformamid |
| DMAP | Dimethylaminopyridin |
| NMP | N-Methylpyrolidon |
| n-H | n-Heptan |
| RT | Raumtemperatur |
| CH₂Cl₂ | Dichlormethan |
| h | Stunden |
| ESI | electron spray ionisation |

Die Erfindung wird durch die nachstehenden Beispiele erläutert.

### Beispiel 1

### 8-[3-(N-(4-trans-Trifluormethylcinnamoylglycyl)-N-methylamino)-2,6-dichlorobenzyloxy]-2-methylchinolin

### a) 2,6-Dichloro-3-nitrobenzylbromid

Zu 2,6-Dichloro-3-nitrotoluol (100 g, 0,48 mol) in Chlorbenzol (400 ml) wurde bei 150°C ein Gemisch aus Dibromhydantoin (70 g, 0,24 mol) und AIBN (5 g) portionsweise zugegeben. Nach 1 h wurde nochmals ein Gemisch aus Dibromhydantoin (35 g, 0,12 mol) und AIBN (2,5 g) zugegeben. Nach weiteren 1,5 h ließ man abkühlen und es wurde EE (500 ml) zugegeben. Dieses Gemisch wurde je 1 mal mit gesättigter Na₂SO₃, Na₂CO₃ und NaCI-Lösung gewaschen, getrocknet (MgSO₄) und eingeengt, wobei die Titelverbindung als amorphes Pulver anfiel.
R_{f} (EE/n-H 1/1) = 0,7 MS (DEI) = 283 (M⁺)

### b) 8-(2,6-Dichlor-3-nitrobenzyloxy)-2-methylchinolin

Zu 8-Hydroxy-2-methylchinolin (5 g, 33,3 mmol) in DMF (65 ml) wurde bei Raumtemperatur Cs₂CO₃ (10,8 g, 33,3 mmol) zugegeben. Nach 30 min wurde 2,6-Dichloro-3-nitro-benzyl-bromid (13g, 45,6 mmol) zugegeben. Nach 18 h wurde H₂O zugegeben und der Niederschlag abgesaugt und mit EE (50 ml) gewaschen, wobei die Titelverbindung als amorphe Substanz anfiel.
R_{f} (EE/n-H 1/2) = 0,3 MS (DEI) = 362 (M)

### c) 8-(2,6-Dichlor-3-aminobenzyloxy)-2-methylchinolin

Zur Titelverbindung des Beispiels 1 b) (4,5 g, 12,4 mmol) in EE (60 ml) wurde SnCl₂·H₂O (15 g, 66,6 mmol) zugegeben und die Suspension auf 70°C erhitzt. Nach 1 h wurde nach Abkühlen auf Raumtemperatur im Vakuum eingeengt und 20 % NaOH Lösung (100 ml) zugegeben und anschließend 3 x mit CH₂Cl₂ extrahiert. Die vereinten organischen Phasen wurden über CaCl₂ getrocknet und eingeengt.
R_{f} (EE/n-H 1/1) = 0,4 MS (FAB) = 333 (M+1)

### d) 8-(2,6-Dichloro-3-phthaloylglycylamino-benzyloxy)-2-methylchinolin

Zur Titelverbindung des Beispiels 1 c) (3,2 g, 10 mmol) und DMAP (1,2 g, 10 mmol) in NMP (30 ml) und Pyridin (10 ml) wurde Phthaloylglycylchlorid (3,4 g, 15 mmol) zugegeben. Die Mischung wurde 1,5 h auf 50°C erhitzt, auf 0°C gekühlt und H₂O (30 ml) zugesetzt. Der Niederschlag wurde abgesaugt und mit EE (100 ml) gewaschen, wobei die Titelverbindung als amorphes Pulver anfiel.
R_{f} (EE/n-H 1/1) = 0,2 MS (FAB) = 520 (M+1)

### e) 8-[3-(N-Phthaloylglycyl-N-methylamino)-2,6-dichlorobenzyloxy]-2-methylchinolin

Zur Titelverbindung des Beispiels 1 d) (3,7 g, 7,1 mmol) in DMF (40 ml) wurde bei 0°C Natriumhydrid (313 mg, einer 60 % Suspension; ~ 8 mmol) zugegeben. Nach 30 min wurde Methyljodid (0,5 ml, 0,8 mmol) zugespritzt. Anschließend wurde die Kühlung entfernt und nach 1 h wieder auf 0°C gekühlt und H₂O (75 ml) zugegeben. Die Titelverbindung wurde abgesaugt und mit kaltem CH₃OH (30 ml) gewaschen.
R_{f} (EE/n-H 1/1) = 0,2 MS (FAB) = 534 (M+1)

### f) 8-[3-(N-Glycyl-N-methylamino)-2,6-dichlorobenzyloxy]-2-methylchinolin

Die Titelverbindung des Beispiels 1 e) (1,5 g, 2,8 mmol) und Hydrazinhydrat (0,54 ml, 11,2 mmol) in Ethanol (60 ml) wurden 12 h bei Raumtemperatur gerührt. Nun wurde eingeengt und CH₂Cl₂ (40 ml) zugegeben und filtriert und der feste Rückstand mit CH₂Cl₂ (40 ml) gewaschen. Einengen der CH₂Cl₂-Lösung lieferte die Titelverbindung als blaßgelben Schaum.
R_{f} (EE/CH₃OH 1/1) = 0,25 MS (FAB) = 404 (M+1)

### g) trans-4-Trifluormethylzimtsäurechlorid

Zu 4-Trifluormethyl-E-zimtsäure (1 g, 4,6 mmol) und Pyridin (375 µl, 4,6 mmol) in trockenem CH₂Cl₂ (11 ml) wurde bei 0°C Thionylchlorid (335 µl, 4,6 mmol) zugegeben. Anschließend wurde 1 h ohne Kühlung gerührt, wieder auf 0°C gekühlt und unter Feuchtigkeitsausschluß filtriert.
Das Filtrat (10 ml) enthielt die Titelverbindung und wurde in Aliquoten für den nächsten Reaktionsschritt verwendet.

### h) 8-[3-(N-(4-trans-Trifluormethylcinnamoylglycyl)-N-methylamino)-2,6-dichlorobenzyloxy]-2-methylchinolin

Zur Titelverbindung des Beispiels 1 f) (250 mg, 0,6 mmol) in CH₂Cl₂ (3 ml) wurde ein Aliquot der Lösung der Titelverbindung des Beispiels 1 g) (2 ml, 1,5 eq, 0,9 mmol) bei Raumtemperatur zugegeben. Nach 18 h wurde gesättigte Na₂CO₃-Lösung (10 ml) zugegeben und 3 mal mit CH₂Cl₂ (3 x 20 ml) extrahiert. Die organischen Phasen wurden getrocknet (CaCl₂) und eingeengt. Chromatographie an SiO₂ mit EE als Eluens lieferte die Titelverbindung des Beispiels 1 als amorphes Pulver.
R_{f} (EE) = 0,4 MS (ESI) = 602 (M+1)

Analog Beispiel 1 wurden die Verbindungen der Beispiele 2 bis 13 erhalten (Tabellen 1 und 2).

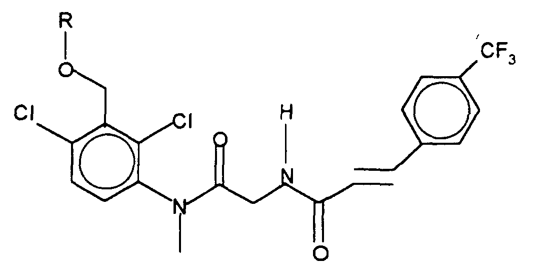

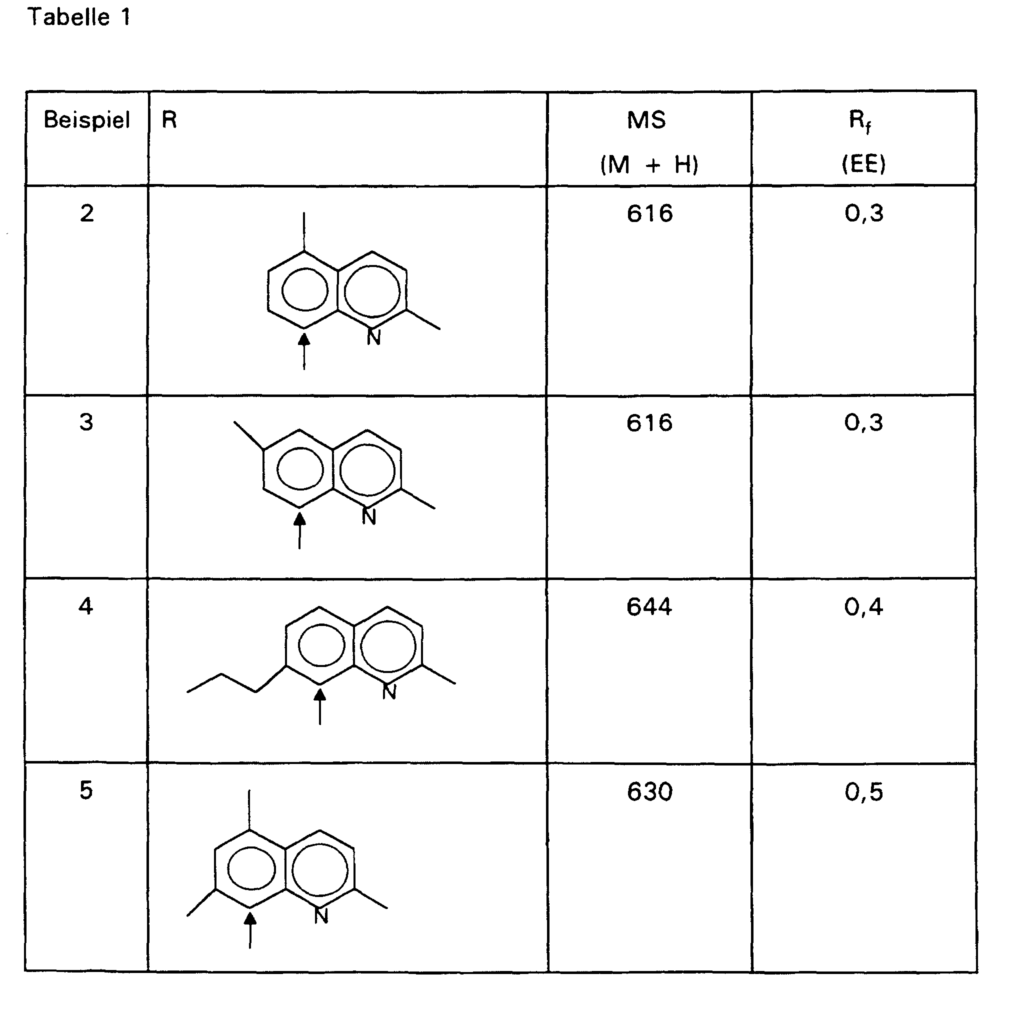

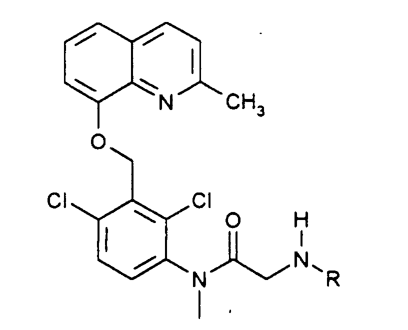

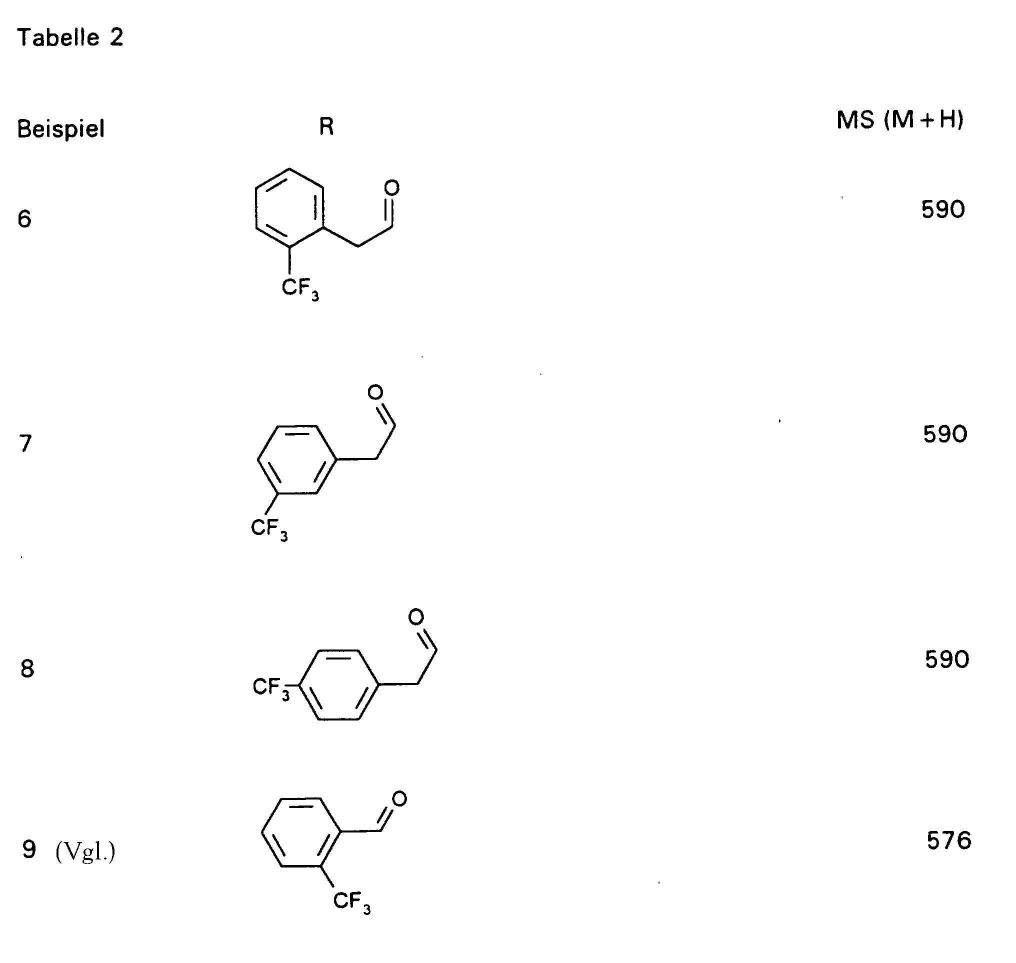

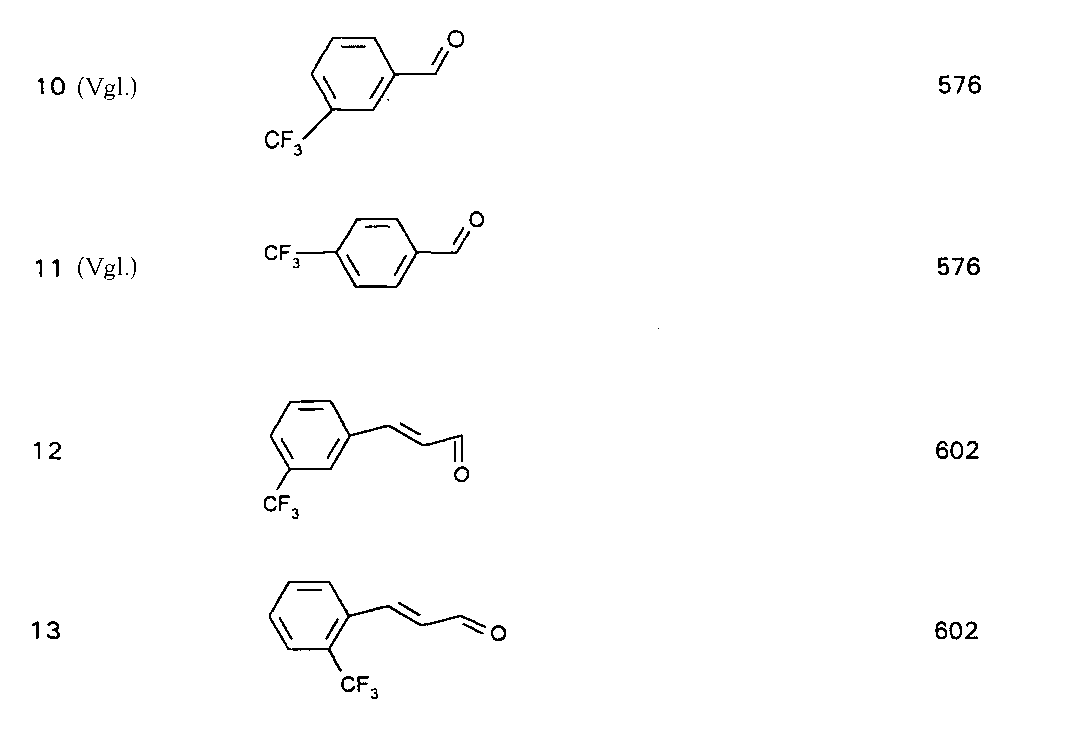

### Vergleichsbeispiel 14

### 8-[2,6-Dichloro-3-(N-(4-trifluormethylbenzyloxy-carbonylamino-acetyl)-N-methylamino)-benzyloxy]-2-methylchinolin

4-Trifluormethylbenzylalkohol (65 mg, 0.37 mmol), 1,1-Carbonyldiimidazol (60 mg, 0.37 mmol) und DMAP (10 mg) werden in Dichlormethan (5 ml) 6 h bei Raumtemperatur gerührt. Dann wurde die Titelverbindung des Beispiels 1f) (150 mg, 0,37 mmol) zugegeben und nach weiteren 18 h wurde Ethylacetat (40 ml) zugegeben. Das Gemisch wurde je 1 mal mit gesättigter Na₂CO₃ und NaCI-Lösung gewaschen, mit MgSO₄ getrocknet und eingeengt. Die Titelverbindung fiel als farbloser Schaum an.
R_{f} (EE) = 0,5 MS (FAB) = 606 (M + 1)

Die Verbindungen der Vergleichbeispiele 15 bis 24 wurden analog Vergleichsbeispiel 14 erhalten (Tabelle 3)

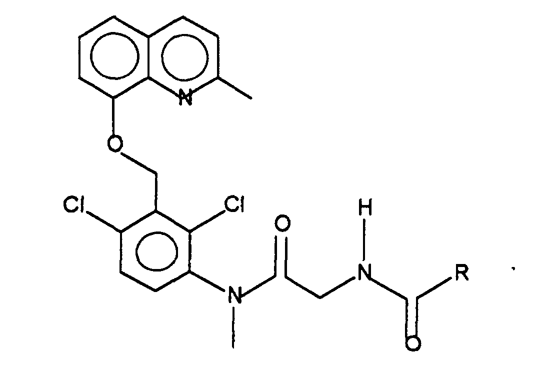

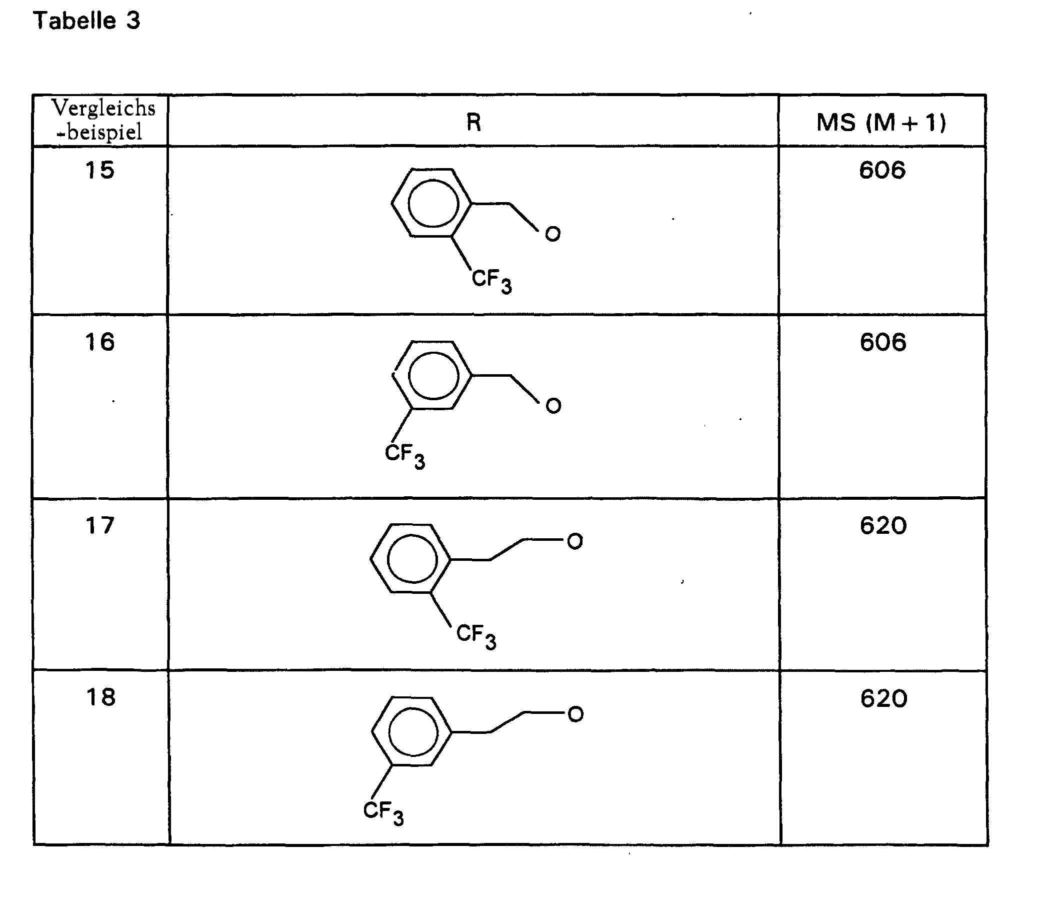

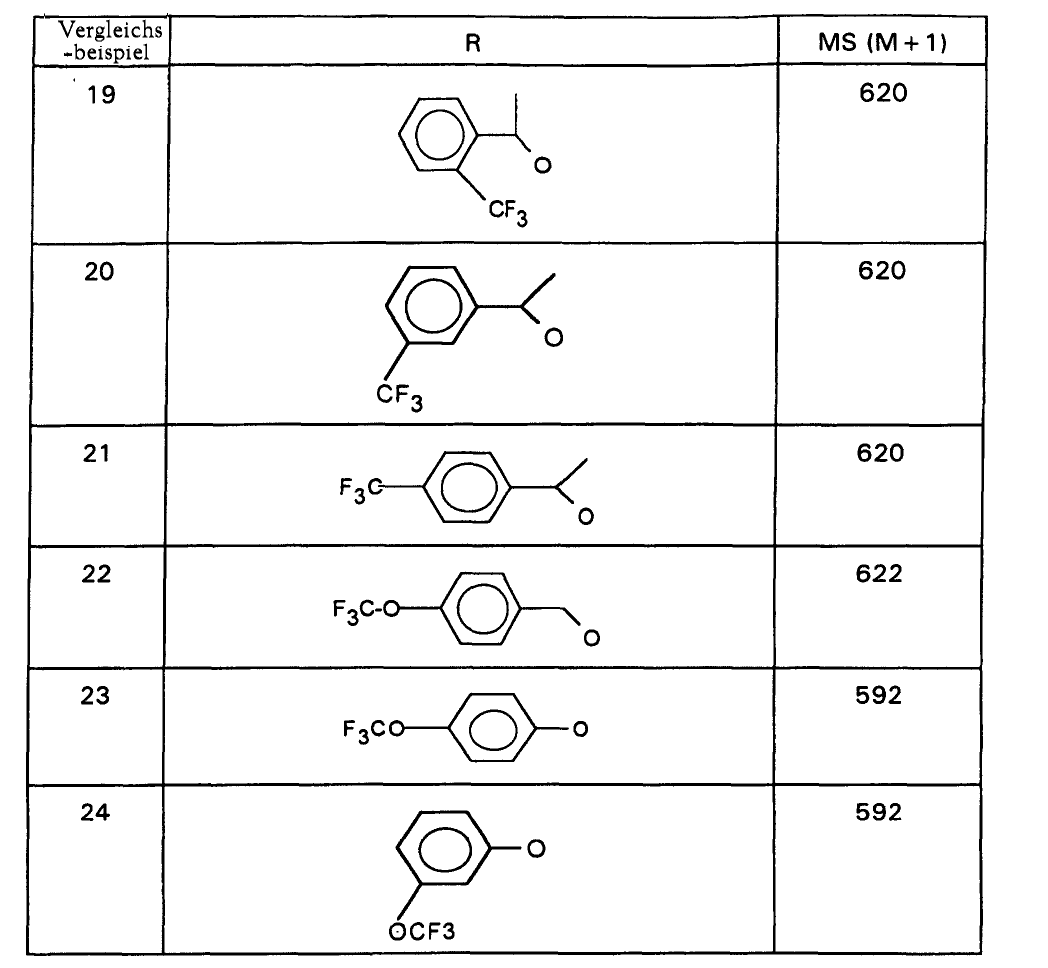

### Vergleichsbeispiel 25

### 8-[3-(N-(4-Trifluormethylphenylureidoacetyl)-N-methylamino)-2,6-dichlorobenzyloxy]-2-methylchinolin

Zur Titelverbindung des Beispiels 1 f) (200 mg, 0.49 mmol) in DME (10 ml) wurde 4-Trifluormethyl-phenylisocyanat (93 mg, 0.49 mmol) gegeben. Nach 3 h bei Raumtemperatur wurde das Lösungsmittel im Vakuum entfernt.

Chromatographie an SiO₂ mit EE als Eluens lieferte die Titelverbindung.
R_{f} (EE) = 0.4 MS (ESI) = 591 (M + 1)

Vergleichsbeispiele 26 bis 28 wurden analog Vergleichsbeispiel 25 erhalten (Tabelle 4).

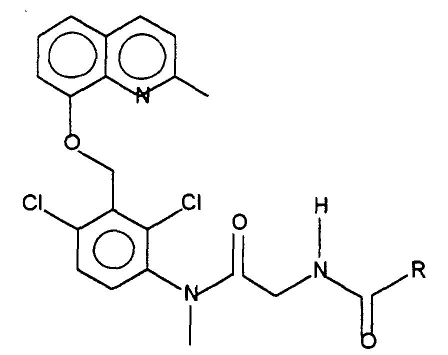

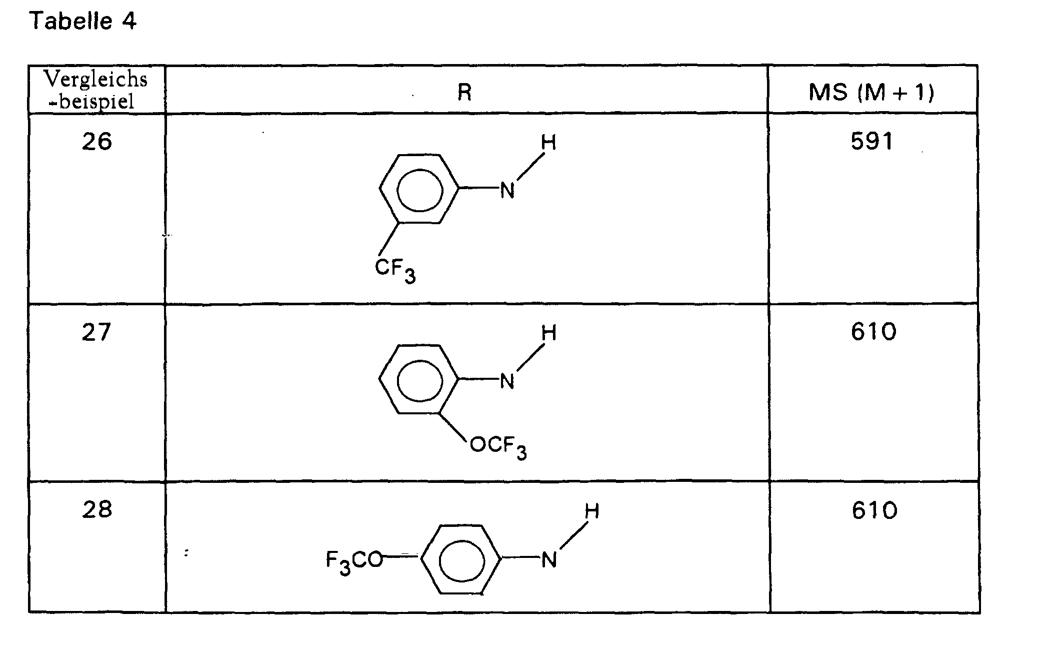

### Vergleichsbeispiel 29

### 8-[3-N-(3-Trifluormethylphenylthioureidoacetyl-N-methylamino)-2,6-dichlorobenzyloxy]-2-methylchinolin

Zur Titelverbindung des Beispiels 1 f) (100 mg, 0.24 mmol) in DME (4 ml) wurde
3-Trifluormethyl-phenylisothiocyanat (50 mg, 0.24 mmol) bei Raumtemperatur zugegeben. Nach 2 Stunden wurde im Vakuum eingeengt und an SiO₂ mit EE als Eluens chromatographiert, wobei die Titelverbindung anfiel.
R_{f} (EE) = 0.5 MS (FAB) = 607 (M + 1 )

## Patentansprüche

1. Heterocyclische Fluoralkylderivate der Formel (I) 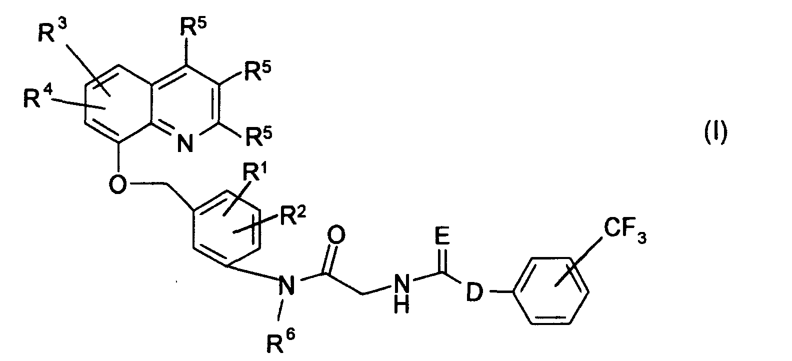 in welcher
R¹ und R² Chlor,
R³ und R⁴ gleich oder verschieden H oder (C₁-C₃)-Alkyl
R⁵ H oder CH₃,
R⁶ (C₁-C₅)-Alkyl,
D (C₁-C₅)-Alkandiyl oder (C₂-C₅)-Alkendiyl, und
E O oder S bedeuten,
sowie deren physiologisch verträglichen Salze.

2. Verfahren zur Herstellung von heterocyclischen Fluoralkylderivaten der Formel (I) gemäß Anspruch 1, **dadurch gekennzeichnet, daß** man
a) eine Verbindung der Formel (II), 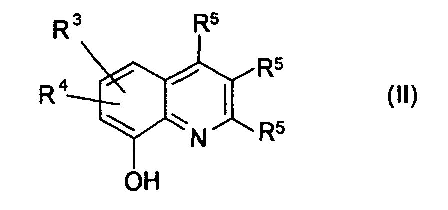 worin R³, R⁴ und R⁵ wie oben in Formel (I) definiert sind, mit Cs₂CO₃ oder K₂CO₃ in einem inerten Lösungsmittel deprotoniert und bei Raumtemperatur mit einer Verbindung der Formel (III) 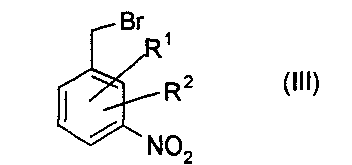 worin R¹ und R² wie oben in Formel (I) definiert sind, umsetzt;
b) die so erhaltene Verbindung der Formel (IV) 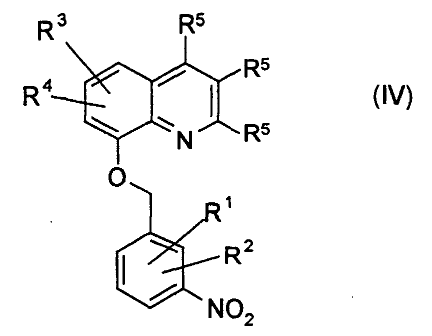 worin R¹, R², R³, R⁴ und R⁵ wie oben in Formel (I) definiert sind, mit Hilfe von Übergangsmetallhalogeniden zu einer Verbindung der Formel (V) reduziert, 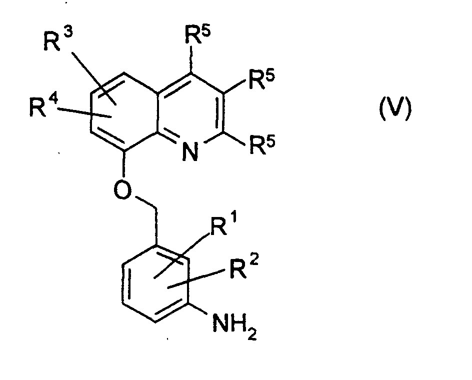 worin R¹, R², R³, R⁴ und R⁵ wie oben in Formel (I) definiert sind;
c) eine Verbindung der Formel (V) mit aktiviertem, geeignet geschützten Glycin (-C(O)-(CH₂) -N(Prot)), in inerten Lösungsmitteln, gegebenenfalls durch Zugabe von DMAP, umsetzt und so eine Verbindung der Formel (VI) erhält, 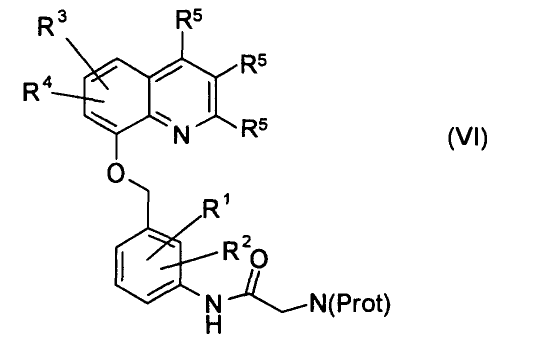 worin R¹, R², R³, R⁴ und R⁵ wie oben in Formel (I) definiert sind, und Prot für eine Aminoschutzgruppe steht,
d) eine Verbindung der Formel (VI) nach erfolgter Einwirkung von Alkalihydriden, Alkalicarbonaten oder Alkoholaten in inerten Lösungsmitteln, gefolgt von einer Behandlung mit R⁶X, wobei R⁶ wie oben in Formel (I) definiert ist und X eine Abgangsgruppe bedeutet, umsetzt, wobei man eine Verbindung der Formel (VII) erhält, 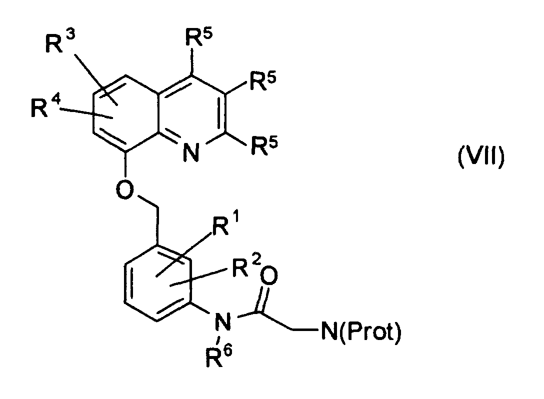 worin R¹, R², R³, R⁴, R⁵ und R⁶ wie oben in Formel (I) und Prot wie oben in Formel (VI) definiert sind;
e) zum Entfernen der Schutzgruppe (Prot) von der Verbindung der Formel (VII), im Falle der Phthaloylgruppe bevorzugt mit Hydrazin in Alkoholen als Lösungsmittel bei Temperaturen zwischen Raumtemperatur und dem Siedepunkt, umsetzt wobei man eine Verbindung der Formel (VIII) erhält, 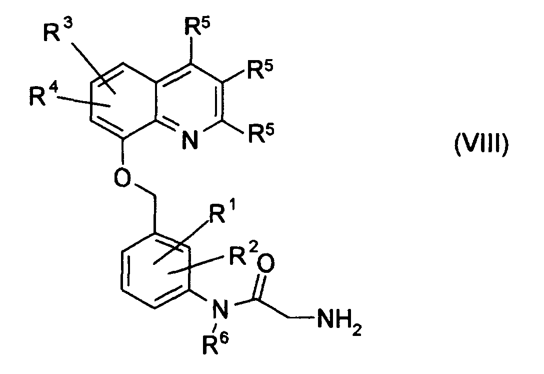 worin R¹, R², R³, R⁴, R⁵ und R⁶ wie oben in Formel (I) definiert sind;
f₁) eine Verbindung der Formel (VIII) mit aktivierten Carbonsäurederivaten der Formel (IX), 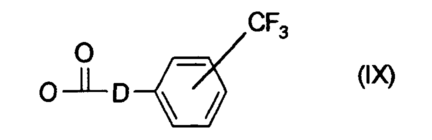 worin D wie oben in Formel (I) definiert ist,
umsetzt, oder
f₂) eine Verbindung der Formel (VIII) mit einem Amin oder einem Alkohol der Formel (X) 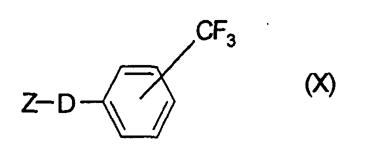 worin D wie oben definiert ist und Z OH oder NH₂ bedeutet, wobei man zunächst jedoch die Verbindung der Formel (VIII) oder (X) mit einer doppelt aktivierten Carbonylverbindung zur Bildung der Harnstoff- bzw. Urethangruppe reagieren läßt, umsetzt oder
f₃) eine Verbindung der Formel (VIII) mit einem entsprechenden Isocyanat oder Isothiocyanat umsetzt, und
g) die erhaltene Verbindung der Formel (I) gegebenenfalls nach bekannten Methoden in ihre physiologisch verträglichen Salze überführt.

3. Verwendung eines Fluoralkylderivates der Formel (I) gemäß Anspruch 1 zur Herstellung eines Arzneimittels zur Behandlung und/oder Prevention von Erkrankungen, die durch Bradykinin oder Bradykinin-analoge Peptide vermittelt, ausgelöst oder unterstützt werden.

4. Heilmittel enthaltend mindestens ein heterocyclisches Fluoralkylderivat der Formel (I) gemäß Anspruch 1.

## Claims

1. A heterocyclic fluoroalkyl derivative of the formula (I) 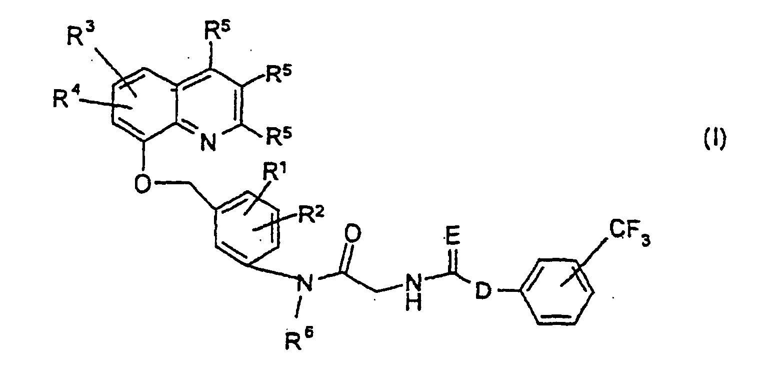 in which
R¹ and R² are chlorine,
R³ and R⁴, identically or differently, are H or (C₁-C₃)-alkyl,
R⁵ is H or CH₃,
R⁶ is (C₁-C₅)-alkyl,
D is (C₁-C₅)-alkanediyl or (C₂-C₅)-alkenediyl, and
E is O or S,
or its physiologically tolerable salts.

2. A process for the preparation of heterocyclic fluoroalkyl derivatives of the formula (I) as claimed in claim 1, which comprises
a) deprotonating a compound of the formula (II), 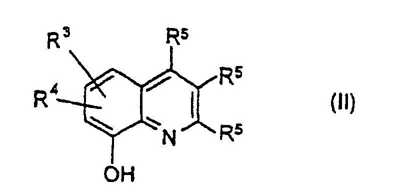 in which R³, R⁴ and R⁵ are as defined in formula (I) above, using Cs₂CO₃ or K₂CO₃ in an inert solvent and reacting at room temperature with a compound of the formula (III) 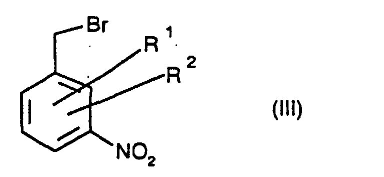 in which R¹ and R² are as defined in formula (I) above;
b) reducing the compound thus obtained of the formula (IV) 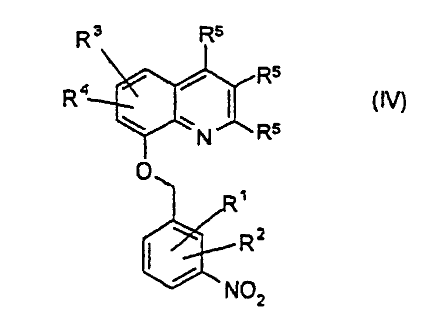 in which R¹, R², R³, R⁴ and R⁵ are as defined in formula (I) above, with the aid of transition metal halides to give a compound of the formula (V) 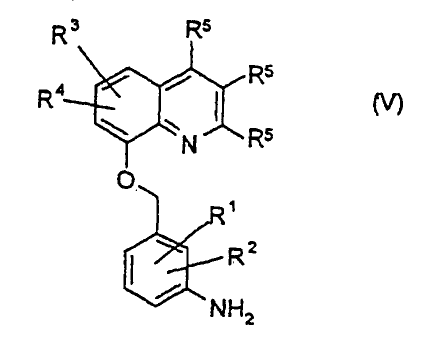 in which R¹, R², R³, R⁴ and R⁵ are as defined in formula (I) above;
c) reacting a compound of the formula (V) with activated, suitably protected glycine (-C(O)-(CH₂) -N(Prot)), in inert solvents, if appropriate by addition of DMAP, and thus obtaining a compound of the formula (VI) 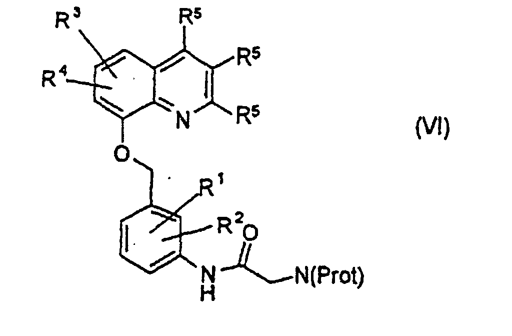 in which R¹, R², R³, R⁴ and R⁵ are as defined in formula (I) above, and Prot is an amino protective group;
d) reacting a compound of the formula (VI), after action of alkali metal hydrides, alkali metal carbonates or alkoxides in inert solvents, followed by a treatment with R⁶X, where R⁶ is as defined in formula (I) above and X is a leaving group,
a compound of the formula (VII) being obtained 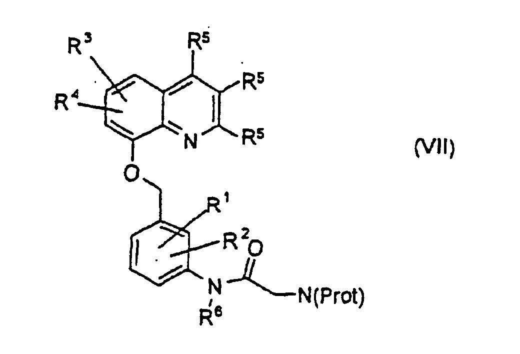 in which R¹, R², R³, R⁴, R⁵ and R⁶ are as defined in formula (I) above and
Prot is as defined in formula (VI) above;
e) to remove the protective group (Prot) from the compound of the formula (VII), in the case of the phthaloyl group preferably reacting with hydrazine in alcohols as solvents at temperatures between room temperature and the boiling point, a compound of the formula (VIII) being obtained 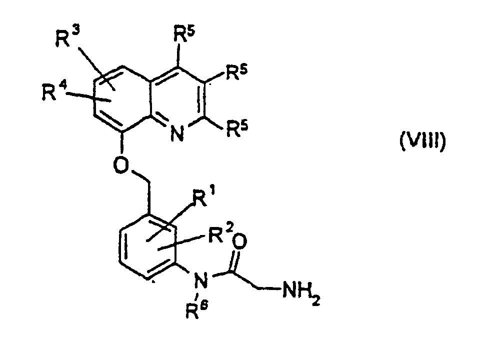 in which R¹, R², R³, R⁴, R⁵ and R⁶ are as defined in formula (I) above;
f₁) reacting a compound of the formula (VIII) with activated carboxylic acid derivatives of the formula (IX) 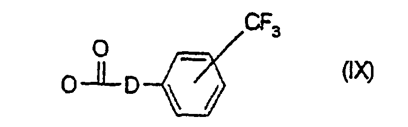 in which D is as defined in formula (I) above,
or
f₂) reacting a compound of the formula (VIII) with an amine or an alcohol of the formula (X) 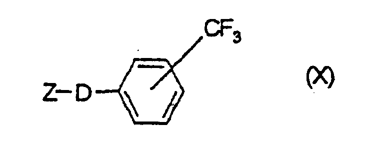 in which D is as defined above and Z is OH or NH₂, the compound of the formula (VIII) or (X), however, first being reacted with a doubly activated carbonyl compound to form the urea or urethane group, or
f₃) reacting a compound of the formula (VIII) with an appropriate isocyanate or isothiocyanate, and
g) if appropriate, converting the compound of the formula (I) obtained into its physiologically tolerable salts by known methods.

3. The use of a fluoroalkyl derivative of the formula (I) as claimed in claim 1 for the preparation of a pharmaceutical for the treatment and/or the prevention of disorders which are mediated, induced or assisted by bradykinin or bradykinin-analogous peptides.

4. A pharmaceutical comprising at least one heterocyclic fluoroalkyl derivative of the formula (I) as claimed in claim 1.

## Revendications

1. Dérivés fluoroalkylés hétérocycliques de formule (I) 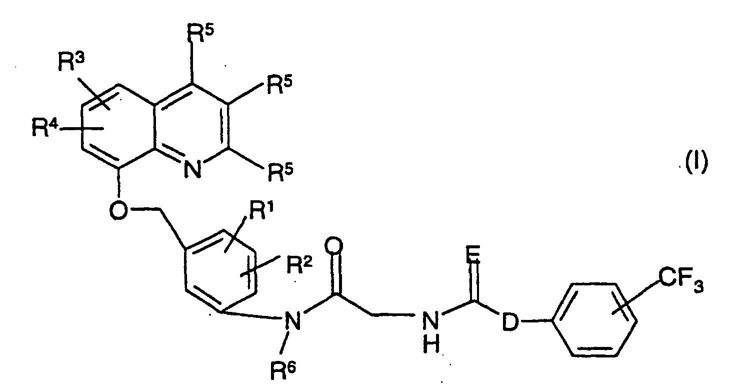 où
R¹ et R² représentent le chlore,
R³ et R⁴ identiques ou différents, représentent H ou alkyle en C₁-C₃,
R⁵ représente H ou CH₃,
R⁶ représente alkyle en C₁-C₅,
D représente alcanediyle en C₁-C₅ ou alcènediyle en C₂-C₅, et
E représente O ou S,
ainsi que leurs sels physiologiquement acceptables.

2. Procédé de préparation de dérivés fluoroalkylés hétérocydiques de formule (I) selon la revendication 1, **caractérisé en ce que**
a) on déprotone un composé de formule (II) 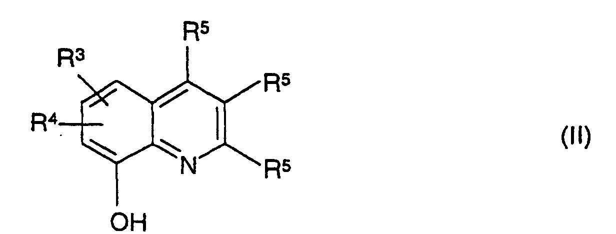 où R³, R⁴ et R⁵ sont définis comme ci-dessus dans la formule (I), avec CS₂CO₃ ou K₂CO₃ dans un solvant inerte et on le fait réagir à la température ambiante avec un composé de formule (III) 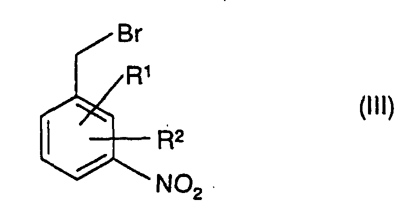 où R¹ et R² sont définis comme ci-dessus dans la formule (I);
b) on réduit le composé ainsi obtenu de formule (IV) 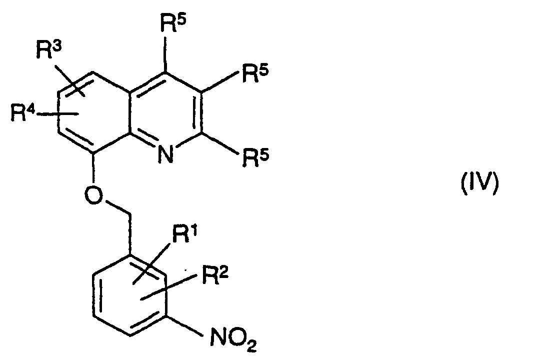 où R¹, R², R³, R⁴ et R⁵ sont définis comme ci-dessus dans la formule (I), à l'aide d'halogénures de métaux de transition, en un composé de formule (V) 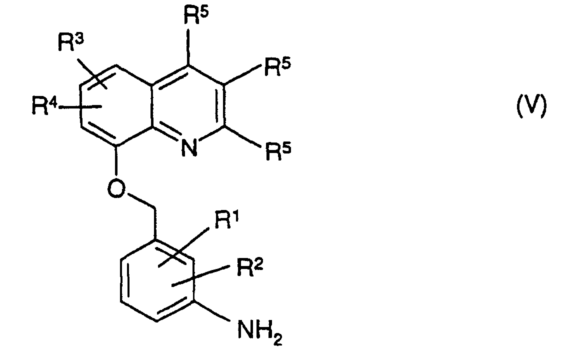 où R¹, R², R³, R⁴ et R⁵ sont définis comme ci-dessus dans la formule (I);
c) on fait réagir un composé de formule (V) avec la glycine activée, protégée de manière appropriée (-C(O)-(CH₂) -N(Prot)), dans des solvants inertes, éventuellement par addition de DMAP, et on obtient ainsi un composé de formule (VI) 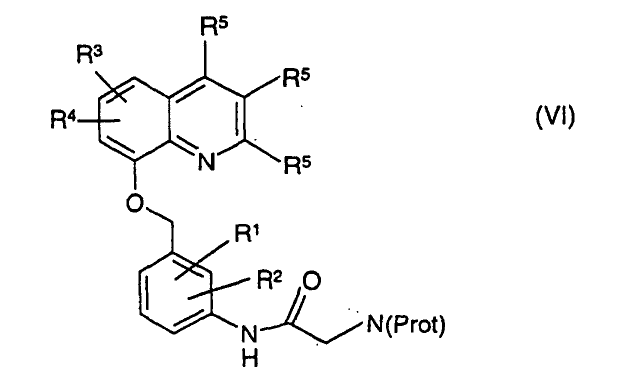 où R¹, R², R³, R⁴ et R⁵ sont définis comme ci-dessus dans la formule (I), et Prot représente un groupe amino-protecteur,
d) on fait réagir un composé de formule (VI) après l'action d'hydrures alcalins, de carbonates alcalins ou d'alcoolates dans des solvants inertes, suivie par un traitement avec R⁶X, où R⁶ est défini comme ci-dessus dans la formule (I) et X représente un groupe partant, de sorte que l'on obtient un composé de formule (VII) 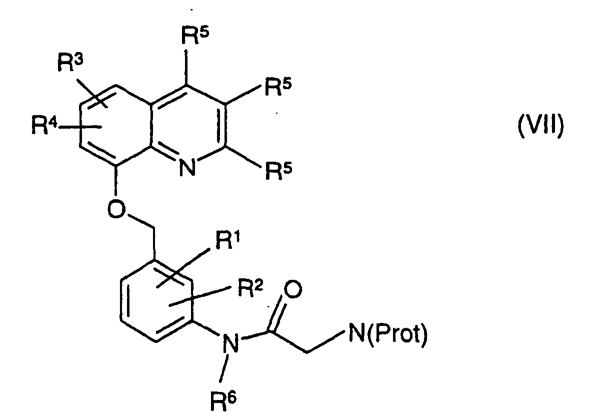 où R¹, R², R³, R⁴, R⁵ et R⁶ sont définis comme ci-dessus dans la formule (I) et Prot est défini comme ci-dessus dans la formule (VI);
e) pour retirer le groupe protecteur (Prot) du composé de formule (VII), dans le cas du groupe phtaloyle, on fait réagir de préférence avec l'hydrazine dans des alcools comme solvant à des températures de la température ambiante au point d'ébullition, de sorte que l'on obtient un composé de formule (VIII) 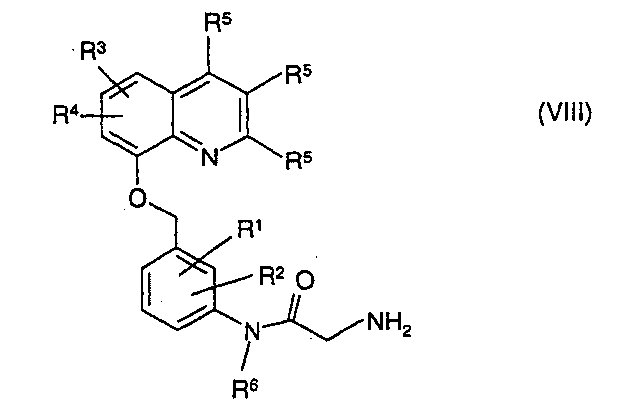 où R¹, R², R³, R⁴, R⁵ et R⁶ sont définis comme ci-dessus dans la formule (I);
f₁) on fait réagir un composé de formule (VIII) avec des dérivés d'acides carboxyliques activés de formule (IX) 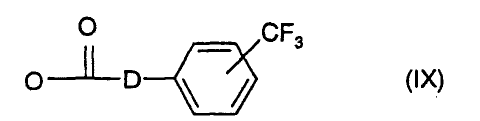 où D est défini comme ci-dessus dans la formule (I),
ou bien
f₂) on fait réagir un composé de formule (VIII) avec une amine ou un alcool de formule (X) 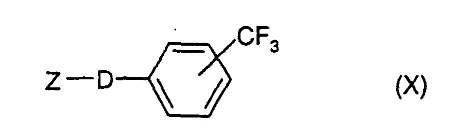 où D est défini comme ci-dessus et Z représente OH ou NH₂, mais en faisant d'abord réagir le composé de formule (VIII) ou (X) avec un composé carbonylé doublement activé pour la formation du groupe urée ou uréthane, ou bien
f₃) on fait réagir un composé de formule (VIII) avec un isocyanate ou isothiocyanate correspondant, et
g) on convertit éventuellement le composé de formule (I) obtenu en ses sels physiologiquement acceptables selon des méthodes connues.

3. Utilisation d'un dérivé fluoroalkylé de formule (I) selon la revendication 1 pour la production d'un médicament pour le traitement et/ou la prévention de maladies qui sont à médiation, déclenchées ou favorisées par la bradykinine ou des peptides analogues à la bradykinine.

4. Médicament contenant au moins un dérivé fluoroalkylé hétérocyclique de formule (I) selon la revendication 1.
